# EUROPÄISCHE PATENTANMELDUNG

(11) **EP 3 188 182 A1**
(43) Veröffentlichungstag der Anmeldung: **05.07.2017**
(21) Anmeldenummer: 15203166.2
(22) Anmeldetag: 30.12.2015
(51) Int. Cl.: G10K 15/02

(54) **AKUSTISCHES SIGNAL**

(71) Anmelder: Meixner, Josip, 1170 Wien (AT)
(72) Erfinder: Meixner, Josip, 1170 Wien (AT)
(74) Vertreter: Babeluk, Michael

(57) **Zusammenfassung**

Die vorliegende Erfindung betrifft ein akustisches Signal zur Beeinflussung der Befindlichkeit von Menschen, bestehend aus einer Aneinanderreihung von Tönen, mit einem weitgehend konstanten Tempo zwischen 50 bpm und 70 bpm, einer geringen Dynamik vorzugsweise in einem Bereich zwischen 50 dB und 60 dB, wobei als Töne ausschließlich Einzeltöne und konsonante Tonzusammensetzungen vorgesehen sind, und wobei ein einfacher und konstanter Rhythmus vorliegt. Dadurch wird eine beruhigende Wirkung erzielt.

## Beschreibung

Die Erfindung betrifft ein akustisches Signal zur Beeinflussung der Befindlichkeit von Menschen, bestehend aus einer Aneinanderreihung von Tönen, mit einem weitgehend konstanten Tempo zwischen 50 bpm und 70 bpm, einer geringen Dynamik in einem Bereich zwischen 50 dB und 60 dB, wobei als Töne ausschließlich Einzeltöne und konsonante Akkorde vorgesehen sind, und wobei ein einfacher und konstanter Rhythmus vorliegt.

Es ist bekannt, dass Musik die Befindlichkeit von Menschen beeinflussen kann. Dies ist typischerweise jedoch ein Nebeneffekt, der dem Unterhaltungsaspekt von Musik untergeordnet ist.

Im Rahmen der vorliegenden Erfindung geht es darum, unabhängig und losgelöst von Unterhaltungsaspekt ein akustisches Signal darzustellen, mit denen gezielt und wirksam auf Menschen gewirkt werden kann. Im Rahmen der vorliegenden Erfindung geht es primär um Ausgleich und Beruhigung, bis hin zur Erzielung eines schläfrigen Gesamtzustandes, der direkt zum Einschlafen der Person führen kann. Dabei werden durchaus auch medizinische Parameter der betreffenden Personen beeinflusst und verändert, wie etwa Puls oder der hormonelle Zustand.

Aufgabe der vorliegenden Erfindung ist es, die psychoakustische Wirkung von Signalen zu optimieren, um eine möglichst weitreichende Beeinflussung von Personen zu erzielen.

Erfindungsgemäß werden diese Aufgaben dadurch gelöst, dass die akustischen Signale aus einer Aneinanderreihung von Tönen bestehen, mit einem weitgehend konstanten Tempo zwischen 50 bpm und 70 bpm, einer geringen Dynamik in einem Bereich zwischen 50 dB und 60 dB, wobei als Töne ausschließlich Einzeltöne und konsonante Tonzusammensetzungen vorgesehen sind, und wobei ein einfacher und konstanter Rhythmus vorliegt.

Es handelt sich also um eine relativ langsame oder getragene Tonfolge, wobei bpm Schläge pro Minute bedeutet, d.h. dass die einzelnen Töne etwa eine Sekunde lang sind, bzw. deren Abstand etwa eine Sekunde beträgt. Wichtig ist, dass das Signal keine dissonanten Tonkombinationen enthält, da diese die beruhigende Wirkung stören können. Als konsonant werden solche Tonzusammensetzungen bezeichnet, bei denen die Frequenzen der Töne, aus denen sie zusammengesetzt sind, in einem einfachen ganzzahligen Verhältnis stehen.

Als einfacher Rhythmus wird ein solcher bezeichnet, hauptsächlich aus gleich langen Tönen besteht und eine regelmäßige Betonung aufweist. In der Musik würde das beispielsweise einem Viervierteltakt entsprechen, der frei von Synkopen ist und bei dem die Musik mehrheitlich in Viertelnoten gesetzt ist.

Wesentlich für die Wirkung des Signals ist, dass der Pegel gering ist, d.h. dass die Lautstärke knapp oberhalb der Hörschwelle liegt und nur sehr geringe Schwankungen aufweist.

Vorzugsweise ist das Signal so konzipiert, dass das Tempo innerhalb einer Schwankungsbreite von weniger als 2 bpm konstant bleibt. Es gibt also keine merklichen Beschleunigungen oder Verzögerungen im Rhythmus.

Es ist auch von besonderem Vorteil, wenn die Lautstärke innerhalb einer Schwankungsbreite von weniger als 3 dB konstant bleibt. Es liegt also ein relativ konstanter Pegel vor.

In besonders bevorzugter Weise sollte das Signal eine geringe rhythmische Komplexität aufweisen. Die bedeutet insbesondere, dass die Dauer eines überwiegenden Anteils der Töne einer konstanten Grunddauer gleich ist und die übrigen Töne eine Dauer aufweisen, die ein Vielfaches oder die Hälfte dieser Grunddauer besitzen. Das Signal besitzt also eine sehr große rhythmische Regelmäßigkeit.

Es ist günstig, wenn die Töne keine definierbare Melodie bilden. Solche Signale sind besonders wirksam.

Eine weitere bevorzugte Ausführungsvariante der Erfindung sieht vor, dass zwei Untersignale vorliegen, die wiederholt und/oder abwechselnd aneinandergereiht sind. Benennt man die Untersignale beispielsweise mit x und y, so sind das Folgen von beispielsweise x - x - y - y - x - y, oder dgl.

Es ist günstig, wenn das Signal eine Gesamtdauer von mindestens fünf Minuten aufweist.

Insbesondere ist es vorteilhaft, wenn das Signal aus einem rechten Kanal und einem linken Kanal zusammengesetzt ist, und dass der rechte und der linke Kanal abwechselnd betont sind. Dabei ist es von besonderem Vorteil, wenn die Betonung in einem Rhythmus von zwei beats wechselt. Die bedeutet, dass zunächst der linke Kanal einen etwas größeren Pegel aufweist, zwei beats oder etwa zwei Sekunden danach der rechte Kanal usw. Insbesondere kann dies dadurch erfolgen, dass die abwechselnde Betonung des rechten und linken Kanals durch kurze atonale Überlagerungen erfolgt. Atonal bedeutet, dass dem Geräusch keine besondere Frequenz zugeordnet werden kann, wie etwa einem weißen Rauschen. Durch diese Überlagerungen, die streng rhythmisch erfolgen und beim Hörer den Eindruck einer regelmäßig arbeitenden Maschine vermitteln können, was aufgrund der geringen Lautstärke beruhigend wirkt.

Die Wirkung wird insbesondere dadurch begünstigt dass eine Gesamtdauer von mehr als einer Stunde vorliegt.

Die vorliegenden Erfindung betrifft auch ein Verfahren zu Beeinflussung der Befindlichkeit von Menschen, bei dem die Menschen mit einer Aneinanderreihung von Tönen mit einem weitgehend konstanten Tempo zwischen 50 bpm und 70 bpm und einer geringen Dynamik vorzugsweise in einem Bereich zwischen 50 dB und 60 dB beschallt werden, wobei als Töne Einzeltöne und konsonante Tonzusammensetzungen vorgesehen sind, und wobei ein einfacher und konstanter Rhythmus vorliegt.

Insbesondere ist vorgesehen, dass die Beschallung knapp oberhalb der Hörschwelle erfolgt.

In der Folge wird die Erfindung anhand eines in den Figuren erklärten Ausführungsbeispiels näher erläutert. Es zeigen:

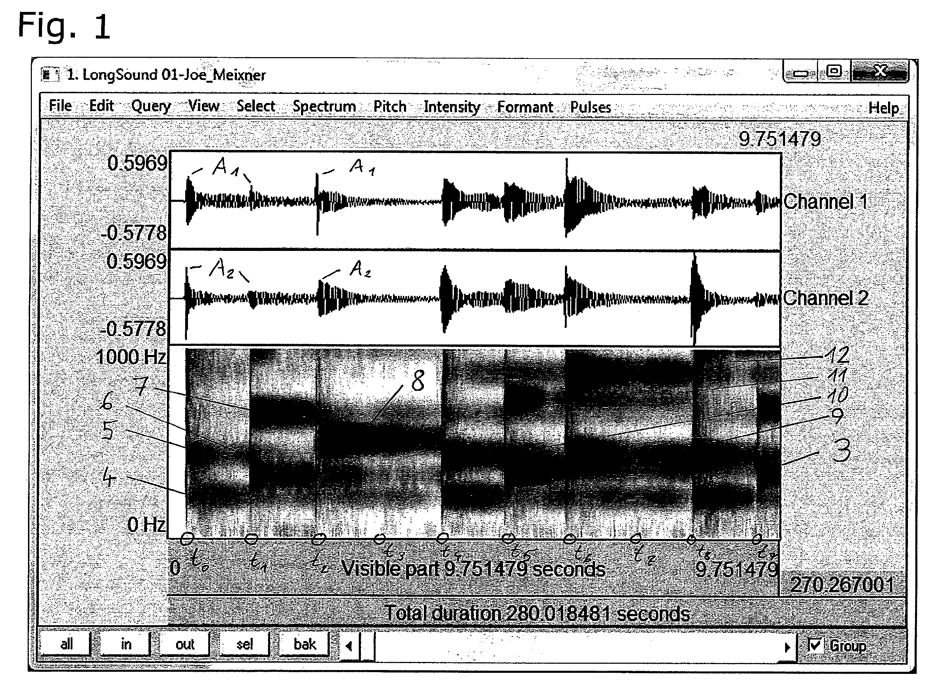

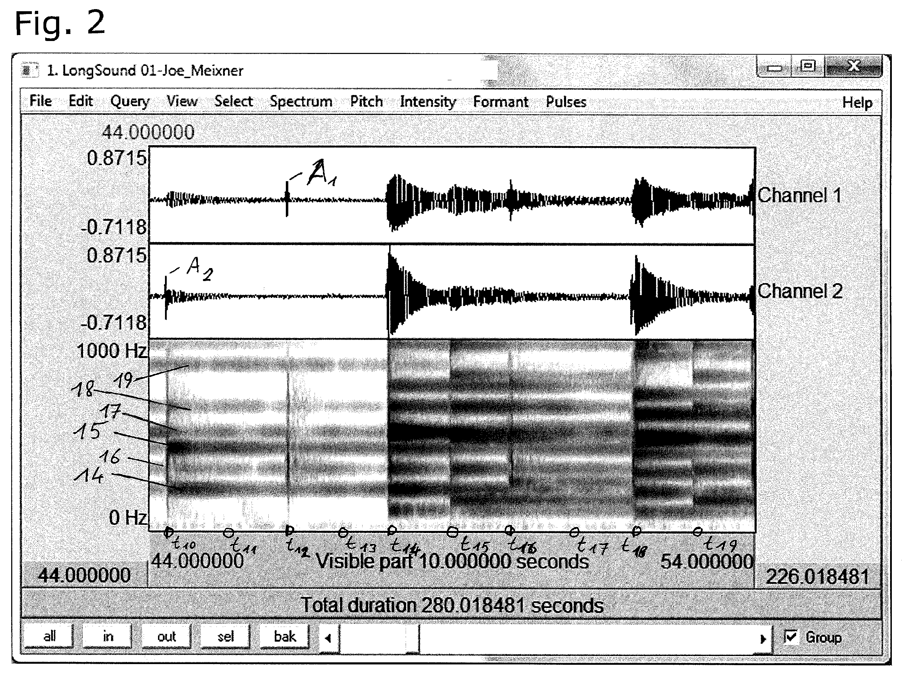

- Fig. 1: und Fig. 2 Diagramme, die die Amplitude und das Spektrum eines erfindungsgemäßen Signals zeigen.

In Fig. 1 ist ein Screenshot eines Spektrogramms über etwa 10 Sekunden eines erfindungsgemäßen Signals dargestellt.

Mit "Channel 1" und "Channel 2" sind der linke und der rechte Kanal bezeichnet, wobei hier die Amplituden ersichtlich sind, während darunter mit 3 ein Spektrum in einem Bereich zwischen 0 Hz und 1.000 Hz dargestellt ist.

In den Zeitpunkten t₀, t₁, t₂, t₃, t₄, t₅, t₆, t₇, t₈, t₉, die regelmäßig im Abstand von etwa einer Sekunde liegen, werden gegebenenfalls die einzelnen Töne erzeugt, die die oben diskutierten beats darstellen. Es ist ersichtlich, dass die Amplituden zu den obigen Zeitpunkten groß ist und danach abnimmt. Zu den Zeitpunkten t₃ und t₇ werden keine Töne erzeugt, die davor erzeugten Töne klingen nach.

Aus dem Spektrum 3 ist ersichtlich, dass im Zeitpunkt t₀, eine Tonzusammensetzung mit zwei Maxima bei 4 und 5 vorliegt, die über den Zeitpunkt t₁ hinaus nachklingt. Die Frequenzen der Töne bei 4 und 5 liegen bei etwa 220 Hz bzw. 440 Hz. Die Frequenzen der beiden Maxima stehen also in einem Verhältnis von 1:2, was einem konsonanten Klang, nämlich einer Oktave entspricht.

Auch im Zeitpunkt t₁ sind zwei Maxima 6 und 7 mit Frquenzen von etwa 330 Hz und 660 Hz erkennbar, die ebenfalls konsonant klingen. Der lang anhaltende Ton 8 im Zeitpunkt t₁ ist ein Einzelton, der zwei Zeiteinheiten bis zum Zeitpunkt t₄ anhält. Im Spektrogramm sind die nachklingenden Töne davor noch ersichtlich.

Im Zeitpunkt t₄ folgt eine Tonzusammensetzung mit zwei Maxima 9 und 10, die von der Tonhöhe her denen bei 4 und 5 entsprechen, wobei zusätzlich Obertöne 11 und 12 ersichtlich sind.

Es ist weiters bemerkenswert, dass zum Zeitpunkt t₀ die Amplitude A₂ des Kanals 2 etwas größer ist als die Amplitude A₁ des Kanals 1. Im Zeitpunkt t₂ ist umgekehrt die Amplitude des Kanals 1 etwas größer ist als die des Kanals 2. Bei t₄ ist wie in t₀ wiederum Kanal 2 dominant. Dieser Wechsel wiederholt sich rhythmisch in t₆, t₈ usw. Dieser Effekt kann durch eine atonale Überlagerung, die sehr kurzzeitig zu den Zeitpunkten t₀, t₂, t₄, t₆, t₈ erfolgt, hervorgerufen oder verstärkt werden. Die Überlagerung ist ein kurzzeitiges Geräusch in der Art eines Schlags, dem keine spezifische Frequenz zuzuordnen ist.

Es ist auch erkennbar, dass die Amplituden A₁ und A₂ in den Zeitpunkten t₀, t₂, t₄, t₆, usw. generell größer sind als in den Zeitpunkten t₁, t₃, t₅, usw.

In Fig. 2 ist ein weiterer Ausschnitt des Signals mit etwa 10 Sekunden Länge analysiert. In den Zeitpunkten t₁₀, t₁₁, t₁₂, t₁₃, t₁₄, t₁₅, t₁₆, t₁₇, t₁₈, t₁₉ erfolgen die Einsätze der einzelnen Töne.

Aus dem Spektrogramm ist ersichtlich, dass von t₁₀ bis t₁₄, eine komplexe Tonzusammensetzung vorliegt, mit dominanten Tönen 14 und 15 mit Frequenzen von 220 Hz bzw. 440 Hz. Weitere Töne 16, 17, 18 und 19 weisen Frequenzen auf, die bei etwa 330 Hz, 660 Hz, 880 Hz und 990 Hz liegen.

Auch hier ist die Dominanz der Amplituden A₁ des ersten Kanals in den Zeitpunkten t₁₂, t₁₆ und der Amplituden A₂ des zweiten Kanals in den Zeitpunkten t₁₀, t₁₄, und t₁₈ zu erkennen.

Durch diese besonderen Eigenschaften, die nicht nur lokal über begrenzte Zeit auftreten, sondern über die gesamte Dauer des Signals, also beispielsweise eine Stunde, wird die besondere Wirkung erzielt.

## Patentansprüche

1. Akustisches Signal zur Beeinflussung der Befindlichkeit von Menschen, bestehend aus einer Aneinanderreihung von Tönen, mit einem weitgehend konstanten Tempo zwischen 50 bpm und 70 bpm, einer geringen Dynamik vorzugsweise in einem Bereich zwischen 50 dB und 60 dB, wobei als Töne ausschließlich Einzeltöne und konsonante Tonzusammensetzungen vorgesehen sind, und wobei ein einfacher und konstanter Rhythmus vorliegt.

2. Signal nach Anspruch 1, **dadurch gekennzeichnet, dass** das Tempo innerhalb einer Schwankungsbreite von weniger als 2 bpm konstant bleibt.

3. Signal nach einem der Ansprüche 1 oder 2, **dadurch gekennzeichnet, dass** die Lautstärke innerhalb einer Schwankungsbreite von weniger als 3 dB konstant bleibt.

4. Signal nach einem der Ansprüche 1 bis 3, **dadurch gekennzeichnet, dass** die Dauer eines überwiegenden Anteils der Töne einer konstanten Grunddauer gleich ist und die übrigen Töne eine Dauer aufweisen, die ein Vielfaches oder die Hälfte dieser Grunddauer besitzen.

5. Signal nach einem der Ansprüche 1 bis 4, **dadurch gekennzeichnet, dass** die Töne keine definierbare Melodie bilden.

6. Signal nach einem der Ansprüche 1 bis 5, **dadurch gekennzeichnet, dass** zwei Untersignale vorliegen, die wiederholt und/oder abwechselnd aneinandergereiht sind.

7. Signal nach einem der Ansprüche 1 bis 6, **dadurch gekennzeichnet, dass** das Signal eine Gesamtdauer von mindestens fünf Minuten aufweist.

8. Signal nach einem der Ansprüche 1 bis 6, **dadurch gekennzeichnet, dass** das Signal aus einem rechten Kanal und einem linken Kanal zusammengesetzt ist, und dass der rechte und der linke Kanal abwechselnd betont sind.

9. Signal nach Anspruch 8, **dadurch gekennzeichnet, dass** die Betonung in einem Rhythmus von zwei beats wechselt.

10. Signal nach einem der Ansprüche 8 oder 9, **dadurch gekennzeichnet, dass** die abwechselnde Betonung des rechten und linken Kanals durch kurze atonale Überlagerungen erfolgt.

11. Anordnung von mehreren Signalen nach einem Ansprüche 1 bis 9, **dadurch gekennzeichnet, dass** eine Gesamtdauer von mehr als einer Stunde vorliegt.

12. Verfahren zu Beeinflussung der Befindlichkeit von Menschen, bei dem die Menschen mit einer Aneinanderreihung von Tönen mit einem weitgehend konstanten Tempo zwischen 50 bpm und 70 bpm und einer geringen Dynamik vorzugsweise in einem Bereich zwischen 50 dB und 60 dB beschallt werden, wobei als Töne Einzeltöne und konsonante Tonzusammensetzungen vorgesehen sind, und wobei ein einfacher und konstanter Rhythmus vorliegt.

13. Verfahren nach Anspruch 11, **dadurch gekennzeichnet, dass** die Beschallung knapp oberhalb der Hörschwelle erfolgt.

14. Verfahren nach einem der Ansprüche 11 oder 12, **dadurch gekennzeichnet, dass** die Beschallung eine Gesamtdauer von mindestens einer Stunde aufweist.

15. Verfahren nach einem der Ansprüche 11 bis 13, **dadurch gekennzeichnet, dass** die Beschallung mit zwei Untersignalen erfolgt, die wiederholt und/ oder abwechselnd aneinandergereiht sind.
